Europäisches Patentamt

European Patent Office

Office européen des brevets

(19)

(11) Publication number: **0 152 030**
**A2**

(12) **EUROPEAN PATENT APPLICATION**

(21) Application number: **85101063.7**

(22) Date of filing: **01.02.85**

(51) Int. Cl.⁴: **C 12 N 15/00**
**C 12 P 21/02, C 12 N 1/18**
**C 07 K 15/00**
**//(C12N1/18, C12R1:865)**

(30) Priority: **03.02.84 JP 16952/84**
**12.03.84 JP 46687/84**

(43) Date of publication of application:
**21.08.85 Bulletin 85/34**

(84) Designated Contracting States:
**BE DE FR GB**

(71) Applicant: **Juridical Foundation Japanese Foundation
for Cancer Research**
**37-1, Kami-Ikebukuro 1-chome Toshima-ku
Tokyo(JP)**

(72) Inventor: **Sugano, Haruo**
**4-8-13, Minami-ogikubo
Suginami-ku Tokyo(JP)**

(72) Inventor: **Kuga, Tetsuro**
**3-9-6, Naka-machi
Machida-shi Tokyo(JP)**

(72) Inventor: **Taniguchi, Tadatsugu**
**A-207,19 Mihogaoka
Ibaraki-shi Osaka-fu(JP)**

(72) Inventor: **Yoshida, Mitsuaki**
**3-11-17, Jiyugaoka
Meguro-ku Tokyo(JP)**

(74) Representative: **Vossius Vossius Tauchner Heunemann
Rauh**
**Siebertstrasse 4 P.O. Box 86 07 67
D-8000 München 86(DE)**

(54) Adult T cell leukemia virus antigen polypeptide.

(57) Recombinant plasmids are constructed by inserting a DNA fragment containing the *env* gene of adult T cell leukemia virus into a vector DNA. Microorganisms are transformed with the recombinant plasmid and thereafter cultured to express the gene and to recover the peptide. The antigenic polypeptide encoded by the *env* gene is useful for detection and diagnosis of adult T cell leukemia.

EP 0 152 030 A2

VOSSIUS · VOSSIUS · TAUCHNER · HEUNEMANN · RAUH
PATENTANWÄLTE

**0152030**

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our Ref: T 549 EP
Case: 386-EPC
Juridical Foundation, Japanese                    February 1, 1985
Foundation for Cancer Research

## ADULT T CELL LEUKEMIA VIRUS ANTIGEN POLYPEPTIDE

The present invention relates to an adult T cell leukemia virus antigen polypeptide encoded by env gene, a recombinant plasmid wherein a DNA fragment coding for the peptide is incorporated, a microorganism containing the plasmid, and a process for producing the adult T cell leukemia virus antigen polypeptide encoded by the env gene using the microorganism.

Adult T cell leukemia virus (hereinafter referred to as ATLV), which is a synonym of human T cell leukemia virus (HTLV), is a C-type retrovirus isolated from patients with adult T cell leukemia (hereinafter referred to as ATL) [Yoshida et al.: Proc. Natl. Acad. Sci., USA, 79, 2031-2036 (1982)]. There are numerous reports that ATL patients have a poor prognosis and that efficacious treatment does not exist leading to a 50% mortality rate within 10 months.

In recent years, an antibody which reacts specifically with cultured MT-1 cells derived from ATL has been shown to exist in the serum of ATL patients [Hinuma et al., Proc. Natl. Acad. Sci., USA, 78, 6476-6480 (1981)]. The existence of this antibody has been confirmed subsequently in most ATL patients and the corresponding antigens are called ATL-associated antigen (hereinafter referred to as ATLA). It has been found that the antibody specific for ATLA (hereinafter referred to as Anti-ATLA antibody) exists in 25% of normal, healthy adults in areas with a high incidence of ATL. It has also been shown that the distribution of cases possessing the anti-ATLA antibody corresponds to the regions with high ATL incidence. Furthermore, it has been shown that ATLA is mainly the antigen of this retrovirus. The existence of ATLV genome in the peripheral blood lymphocytes of patients has been established. ATLV has also been detected by culturing the lymphocytes of normal people who are positive to anti-ATLA antibody.

There is a very close correlation between ATL and ATLV, and ATLV is considered to be the causative virus of ATL. Though the route by which infection occurs is still unknown, it has been proved that transfusion of blood is one of the routes. As 25% of healthy people in areas with a high incidence of ATL are anti-ATLA antibody positive, the likelihood that they are carriers of ATLV is extremely high, which means that they must be avoided as blood donors for transfusion.

Therefore, detection of the presence of anti-ATLA antibody will enable avoidance of transfusion from the carriers and early ATL detection. At the present time, detection of anti-ATLA antibody is conducted using acetone fixed slides of cultured cells derived from ATL. However, a simpler, faster method of anti-ATLA antibody detection and earlier ATL diagnosis are desirable.

The present inventors have studied about a method for providing one of the ATLA which is useful for detection of anti-ATLA antibody and prevention of ATLV infection in a large amount and at low cost. As the result, it has now been found that an ATLV antigen polypeptide encoded by the env gene of ATLV genome can be accumulated in a large amount by culturing a yeast containing a recombinant DNA which is obtained by incorporating the env gene into a vector DNA using recombinant DNA techniques.

It has already been reported that one of the products derived from the env gene is a glycosylated protein (gp 62) having a molecular weight of 62,000 [Hattori et al.: Gann, 74, 790-793 (1983)] and the entire DNA sequence of ATLV was determined by the present inventors [Japanese Patent Application No. 214287/82, Proc. Natl. Acad. Sci., USA, 80, 3618-3622 (1983)].

The present invention provides a recombinant plasmid wherein a DNA fragment containing the env gene of ATLV is incorporated, a microorganism containing the plasmid, and a process for producing an ATLV antigen peptide (product derived from the env gene) using the microorganism. The production of ATLV antigen polypeptide encoded by the env gene using a microorganism was achieved first by the present invention.

The ATLV surface antigen protein produced by the present invention contains the entire protein of the surface antigen and so all the antigenic determinants present plurally on the surface antigen. Therefore, it is considered to be useful as an antigen for diagnosis and vaccination.

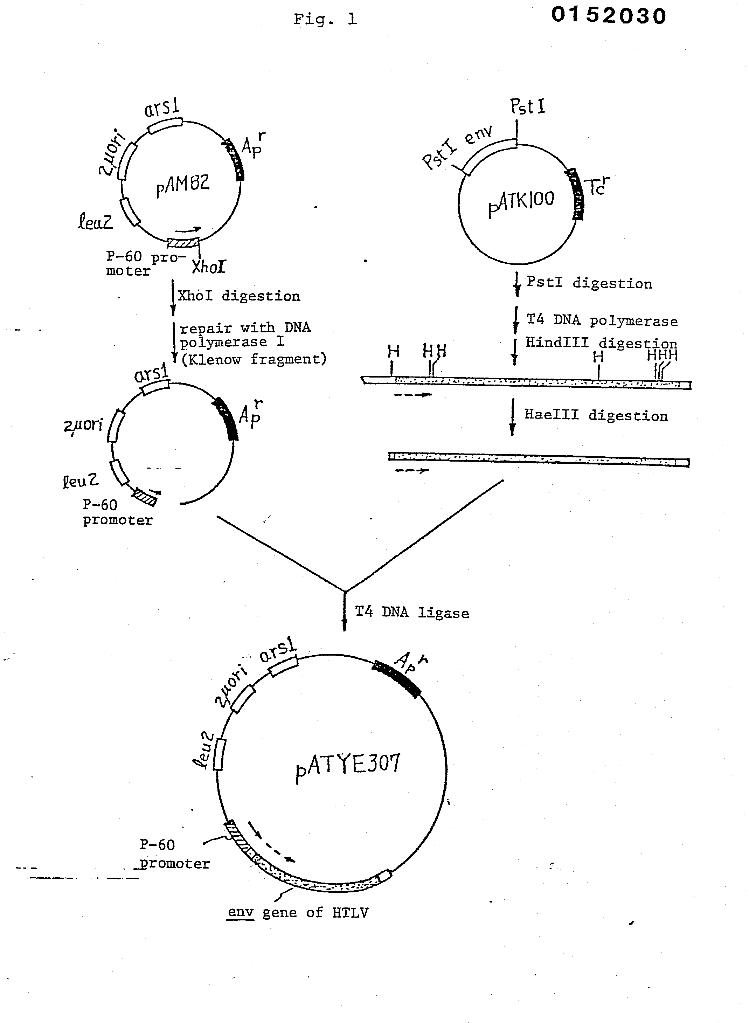

Fig. 1 is a flow sheet showing the construction of plasmid pATYE307. In the drawing, H refers to HaeIII site, the arrow → refers to the transcription direction of P-60 and the arrow ---→ refers to the transcription direction of the env gene.

The present invention provides a recombinant plasmid wherein a DNA fragment containing the env gene of ATLV is incorporated, a microorganism containing the plasmid, and a process for producing an ATLV antigen peptide (product derived from the env gene) using the microorganism.

The construction of the recombinant plasmid of the present invention is carried out in the following manner.

The recombinant plasmid can be constructed by incorporating a DNA coding for the env gene of ATLV into a vector DNA using recombinant DNA techniques.

As the DNA coding for the env gene of ATLV, for example, pATK100 cloned by Seiki et al. [Seiki et al., Proc. Natl. Acad. Sci., USA, 80, 3613-3622 (1983)] can be used. The genome of ATLV consists of the LTR at both ends and of at least four genes, namely, gag, pol, env and pX. Since the protein encoded by the env gene is known to be a surface antigen expressed by the infection of ATLV, it is expected that the protein is useful for the diagnosis of ATLV infections, prevention of ATLV infections and treatment of leukemia caused by ATLV. It is also possible to use the protein itself as a vaccine.

pATK100 is a clone containing the entire env gene and can be used as a source of the DNA fragment containing the env gene.

Any vector DNA can be utilized, provided that the inserted desired DNA can be expressed in a microorganism. A plasmid which has an appropriate promoter such as those derived from 3-phosphoglycerate kinase (PGK), tryptophan (TRP1), alcohol dehydrogenase I (ADHI), galactose kinase (GAL1), glycerolaldehyde-3-phosphate dehydrogenase (GAPDH) and p-60 of yeast and in which a desired DNA can be inserted downstream from the promoter is preferably used.

As the plasmid, pYeHBs, pMA36, pMA56, pAM82 and the like are employable. It is preferable to use pAM82, which can be prepared by the method described in Miyanohara et al, Proc. Natl. Acad. Sci., USA, 80, 1-5 (1983). pAM82 is a plasmid which has the replication initiation site and ampicillin resistance (Ap$^R$) gene derived from pBR322, the arsl gene and leu2 gene derived from yeast and the replication initiation site of 2μ DNA and which is capable of

replicating autonomously in <u>Escherichia</u> <u>coli</u> and yeast and of being identified in the plasmid-carrying strain.

Further, pAM82 has the promoter region of the polypeptide (P-60) gene of repressible acid phosphatase derived from yeast, and therefore, a foreign DNA can be expressed by inserting a gene having a translation initiation site into the plasmid at the unique XhoI site.

Recombination of a DNA containing the <u>env</u> gene of ATLV, for example, a DNA from pATK100 and a vector DNA, for example, pAM82 can be carried out using general recombinant DNA techniques in which both DNAs are digested with restriction enzymes followed by ligation using T4 DNA ligase. Ligation may be conducted by a method employing fill-in reaction with DNA polymerase I·Klenow fragment or a method using a DNA linker.

In the case of pATK100 and pAM82 mentioned as examples, as shown in Fig. 1, recombinant plasmid pATYE307 can be constructed by digesting the <u>env</u> gene of pATK100 with restriction enzyme PstI, changing the ends to blunt ends with T4 DNA polymerase and inserting the DNA fragment obtained by a further digestion with HindIII and HaeIII into an XhoI site of pAM82.

The reaction conditions necessary for the above-described preparation of the recombinant plasmid are generally as follows. DNA digestion with restriction enzymes is normally carried out by 15 minutes - 24 hours digestion of 0.1 - 100 µg of DNA, at 18 - 42°C, preferably 32 - 38°C, using 0.1 - 300 units, preferably 1 - 3 units, of restriction enzyme per 1 µg of DNA in 2 - 200 mM, preferably 10 - 40 mM Tris-HCl (pH 6.0 - 9.5, preferably pH 7.0 - 8.0) 1 - 150 mM NaCl and 2 - 20 mM, preferably 5 - 10 mM $MgCl_2$. The reaction is terminated by heating at 55 - 75°C, preferably 63 - 70°C, for 5 - 30 minutes. The restriction enzymes may be inactivated by reagents such as phenol and diethylpyrocarbonate. Ligation of DNA fragments is conducted at 1 - 37°C, preferably 3 - 20°C, for 15 minutes to 72 hours, preferably 2 - 20 hours using 0.1 - 10 units of T4 DNA ligase in 2 - 200 mM, preferably 10 - 70 mM Tris-HCl (pH 6.0 - 9.5, preferably pH 7.0 - 8.0), 2 - 20 mM, preferably 5 - 10 mM $MgCl_2$, 0.1 - 10 mM, preferably 0.5 - 2 mM ATP and 1 - 50 mM, preferably 5 - 10 mM dithiothreitol.

Purification of the DNA fragments, recombinant plasmids, etc. is carried out by agarose gel electrophoresis.

ATLV antigen polypeptide encoded by the env gene is obtained by culturing a transformant obtained by introducing a recombinant plasmid such as pATYE307 into a microorganism.

It is desirable to use yeast as the host microorganism and Saccharomyces cerevisiae AH22 (a, leu2, his4, canl, cir$^+$) [Ito, H., et al., J. Bacteriol., 153, 163 - 168 (1983)] is preferably used.

Transformation is carried out in accordance with the method of H. Ito et al. [J. Bacteriol., 153, 163-168 (1983)]. Transformants can be obtained as leucine non-requiring strains in the case of pATYE307.

Expression of the ATLV antigen polypeptide by the transformant is confirmed, for example, by culturing the strain in the Burkholder minimal medium or its improved medium, disrupting the cells, subjecting the disrupted cells to extraction with a phosphate buffer solution containing phenylmethylsulfonylfluoride, subjecting the extract to gel electrophoresis in the presence of SDS and detecting the ATLV coat protein produced in the yeast by the method of Towbin et al. [Towbin, H., et al., Proc. Natl. Acad. Sci., USA, 76, 4350-4354 (1979)].--The polypeptide detected in this way is ascertained to give specific immune agglutination with patient serum, which indicates that it has the properties of ATLV antigen peptide.

Isolation of the plasmids from the yeast is carried out in accordance with the method of K. Struhl. et al.: Proc. Natl. Acad. Sci., USA, 76, 1035 (1979). A transformant carrying pATYE307 has been deposited with the American Type Culture Collection, U.S.A. under the Budapest Treaty as Saccharomyces cerevisiae CI11, ATCC 20697.

Certain specific embodiments of the invention are illustrated by the following representative examples.

Example 1

200 µg of pATK100 [Seiki, M., et al., Proc. Natl. Acad. Sci., USA, 80, 3618-3622] was dissolved in 1000 µℓ of a buffer solution consisting of 50 mM NaCl, 10 mM Tris-HCl (pH 7.5), 7 mM $MgCl_2$ and 6 mM 2-mercaptoethanol. 200 units of restriction enzyme PstI (Product of Takara Shuzo Co., the restriction enzymes hereinafter are all products of Takara Shuzo Co., unless otherwise specified) was added and reaction was carried out at 37°C for 1 hour. The reaction product was subjected to 0.9% agarose gel electrophoresis and staining with 1 µg/mℓ ethidium bromide to detect DNA fragments. A DNA fragment of 2,425 base pairs (referred to as bp hereinafter) containing the env gene was recovered. 41 µg of the DNA fragment was added to 300 µℓ of a solution consisting of 30 mM Tris-acetate (pH 7.9), 66 mM potassium acetate, 10 mM magnesium acetate, 0.5 mM dithiothreitol, 0.1 mM dATP, 0.1 mM dTTP, 0.1 mM dGTP and 0.1 mM dCTP. 4 units of T4 DNA polymerase (product of Takara Shuzo Co.) was added and reaction was carried out at 37°C for 10 minutes. By this reaction, the cohesive end of the DNA fragment formed by the cleavage with PstI was repaired to a blunt end.

40 µg of the DNA fragment was dissolved in 200 µℓ of a buffer solution consisting of 50 mM NaCl, 10 mM Tris-HCl (pH 7.5), 7 mM $MgCl_2$ and 6 mM 2-mercaptoethanol. Then, 50 units of restriction enzyme HindIII was added and reaction was carried out at 37°C for 1 hour to obtain a DNA fragment of 1,743 bp containing the env gene. 27 μg of the DNA fragment of 1,743 bp was dissolved in 250 μℓ of a buffer solution consisting of 50 mM NaCl, 10 mM Tris-HCl (pH 7.5), 7 mM MgCl₂ and 6 mM 2-mercaptoethanol. Then, 42 units of restriction enzyme HaeIII was added and reaction was carried out at 37°C for 25 minutes. The reaction product was subjected to 1.4% agarose gel electrophoresis and staining with 1 μg/mℓ ethidium bromide to detect DNA fragments, and a DNA fragment of 1,590 bp containing the env gene was recovered.

Separately, 3 μg of vector plasmid pAM82 (Miyanohara, A., et al., Proc. Natl. Acad. Sci., USA, 80, 1-5, 1983) was dissolved in 20 μℓ of a buffer solution consisting of 100 mM NaCl, 10 mM Tris-HCl (pH 7.5), 7 mM MgCl₂ and 6 mM 2-mercaptoethanol. Then, 8 units of restriction enzyme XhoI was added and reaction was carried out at 37°C for 1 hour. The reaction solution containing the DNA fragment was added to 25 μℓ of a solution consisting of 50 mM Tris-HCl (pH 7.8), 5 mM MgCl₂, 10 mM 2-mercaptoethanol, 5 μg/mℓ BSA (bovine serum albumin), 0.1 mM dATP, 0.1 mM dTTP, 0.1 mM dGTP and 0.1 mM dCTP. Then, 3 units of DNA polymerase I (Klenow fragment) was added and reaction was carried out at 22°C for 30 minutes. By the reaction, the cohesive end of the DNA fragment formed by the cleavage with XhoI was repaired to a blunt end and a DNA fragment of about 11,000 bp was obtained.

0.23 μg of the DNA fragment (1,590 bp) derived from pATK100 and 0.84 μg of the DNA fragment (about 11,000 bp) derived from pAM82 were added to 50 μℓ of a solution consisting of 66 mM Tris-HCl (pH 7.5), 6.6 mM MgCl₂, 10 mM ditiothreitol and 0.5 mM ATP. Then, 3 units of T4 DNA ligase (product of Takara Shuzo Co.) was added and ligation reaction was carried out at 4°C for 1 day.

The reaction solution was subjected to phenol extraction to remove proteins and Escherichia coli HB101 [Bolivar et al., Gene 2, 75 (1977)] was transformed using the resulting solution according to the conventional method. Transformants were selected as strains which grow on a medium consisting of L-Broth (10 g/ℓ Bacto-trypton, 5 g/ℓ yeast extract and 5 g/ℓ NaCl) and 25 µg/mℓ ampicillin. A plasmid was isolated from a transformant according to the method described in H.C. Birnboim et al., Nucleic Acids Research 7, 1513 (1979) and named pATYE307.

It was confirmed by the method of Maxam-Gilbert [A.M. Maxam et al.: Proc. Natl. Acad. Sci., USA, 74, 560 (1977)] that pATYE 307 was a recombinant wherein the env gene was incorporated in the same transcription direction as the P-60 promoter of vector plasmid pAM82 and that it had a structure which enables the expression of the env gene of ATLV under the control of the P-60 promoter, as illustrated in the following structure.

```
          ---TGAAACGTATATAAGCGCTGATGTTTTGCTAAGTCGAGG
                   Hogness box
          TTAGTATGGCTTCATCTCTCATGAGAATAAGAACAACC
                    Capping box
          TCGCCCCGCCGATCCCAAAGAAAAAGACCACCAACACC
                       ←—|
P-60 promoter
region

          ATGGGTAAG--------
          |——→
            env structural gene
```

Saccharomyces cerevisiae AH22 (a, leu2, his4, canl, cir⁺) (Ito, H., et al., J. Bacteriol., 153, 163-168, 1983) was transformed with the plasmid pATYE307 by the Li⁺ method (the same reference as mentioned above). Transformants were selected as strains which grow on a medium consisting of 6.7 g/ℓ yeast nitrogen base without amino acids (product of Difco), 20 g/ℓ glucose and 20 µg/mℓ histidine.

A transformant carrying pATYE307 has been deposited with the American Type Culture Collection, U.S.A. as _Saccharomyces_ _cerevisiae_ CI11, ATCC 20697.

Example 2

The transformant CI11 obtained in Example 1 and the L-82-1 strain obtained by introducing pAM82 into AH22 strain were inoculated in an improved Burkholder minimal medium (pH 5.0) which contains the following components in 1ℓ: 20g of glucose, 2g of asparagine, 1.5g of $KH_2PO_4$, 0.33g of $CaCl_2 \cdot 2H_2O$, 0.5g of $MgSO_4 \cdot 7H_2O$, 2g of $(NH_4)_2SO_4$, 0.1 mg of KI, 60 µg of $H_3BO_4$, 30 µg of $MnSO_4$, 300 µg of $ZnSO_4 \cdot 7H_2O$, 40 µg of $CuSO_4 \cdot 5H_2O$, 250 µg of $FeCl_3 \cdot 6H_2O$, 25 µg of $Na_2MoO_4 \cdot 2H_2O$, 200 µg of thiamine, 200 µg of pyridoxine, 200 µg of nicotinic acid, 200 µg of pantothenic acid, 2 µg of biotin, 10 mg of inositol, and 20 mg of histidine. Culturing was carried out at 30°C with shaking at 200 rpm overnight. The harvested cells were washed with sterilized water and suspended in a production medium prepared by reducing the concentration of $KH_2PO_4$ in the improved Burkholder minimal medium to 30 mg/ℓ and adding 1.5g/ℓ KCl thereto to an $OD_{660}$ value of 0.05. Culturing was carried out at 30°C with shaking at 200 rpm for 20 - 24 hours. Harvested cells were washed with sterilized water and suspended in 0.5 mℓ of 50 mM phosphate buffer solution (pH 7.2) containing 1 mM phenylmethylsulfonylfluoride. The cells were disrupted with glass beads to prepare an extract. The extract was subjected to polyacrylamide gel electrophoresis in the presence of 0.1% SDS and Western blotting (protein blotting) by the method of Towbin _et al._ [Towbin, H., _et al._, Proc. Natl. Acad. Sci., USA, 76, 4350-4354 (1979)] to detect ATLV envelope protein produced in the yeast. As first antibody and second antibody in the above method, the serum of an ATL patient and [$^{125}$I] labelled anti-human immune-globulin (product of Amersham) were used, respectively.

As the result, a protein of a molecular weight of about 43,000 was detected as a specific band. In the case where the normal human serum was used as the first antibody, the band was not detected. Further, the band was not detected in the yeast strain L-82-1 transformed with pAM82 used as a vector. Therefore, it is certain that the protein is a product derived from the ATLV envelope protein gene.

WHAT IS CLAIMED IS:

1.    An adult T cell leukemia virus antigen polypeptide encoded by the _env_ gene of adult T cell leukemia virus.

2.    A recombinant plasmid wherein a DNA fragment containing the _env_ gene of adult T cell leukemia virus is incorporated.

3.    The recombinant plasmid according to claim 2, wherein the recombinant plasmid carries the P-60 promoter.

4.    The recombinant plasmid according to claim 3, which is pATYE307.

5.    A process for producing an adult T cell leukemia virus antigen polypeptide which comprises culturing in a medium a microorganism carrying a recombinant plasmid wherein a DNA fragment containing the _env_ gene of adult T cell leukemia virus is incorporated, accumulating adult T cell leukemia virus antigen polypeptide encoded by the _env_ gene in the culture      and recovering the peptide therefrom.

6.    The process according to claim 5, wherein the recombinant plasmid carries the P-60 promoter.

7.    The process according to claim 5, wherein the microorganism belongs to the genus _Saccharomyces_.

8.    The process according to claim 7, wherein the microorganism belongs to _Saccharomyces cerevisiae_.

9.    The process according to claim 5, wherein the recombinant plasmid is pATYE307.

0152030

10.    A microorganism containing a recombinant plasmid wherein a DNA fragment containing the <u>env</u> gene of adult T cell leukemia virus is incorporated.

11.    The microorganism according to claim 10, wherein the recombinant plasmid carries the P-60 promoter.

12.    The microorganism according to claim 11, wherein the microorganism belongs to <u>Saccharomyces cerevisiae</u>.

Fig. 1      0152030

Fig. 1